# FASCICULE DE BREVET EUROPEEN

(11) **EP 0 801 742 B1**
(45) Date de publication et mention de la délivrance du brevet: **13.06.2001**
(21) Numéro de dépôt: 96901022.2
(22) Date de dépôt: 05.01.1996
(51) Int. Cl.: G01N 29/02

(54) **DISPOSITIF DE CONTROLE DE L'ECOULEMENT D'UN LIQUIDE DANS UNE CONDUITE TUBULAIRE ET NOTAMMENT DANS UNE POMPE PERISTALTIQUE**
VORRICHTUNG ZUR DURCHFLUSSKONTROLLE IN EINER RÖHRE INSBESONDERE IN EINER PERISTALTISCHEN PUMPE
DEVICE FOR CONTROLLING A LIQUID FLOW IN A TUBULAR DUCT AND PARTICULARLY IN A PERISTALTIC PUMP

(30) Priorité: 05.01.1995 FR 9500053
(43) Date de publication de la demande: 22.10.1997
(73) Titulaire: DEBIOTECH S.A., 1000 Lausanne 9 (CH)
(72) Inventeur: NEFTEL, Frédéric, CH-1000 Lausanne 9 (CH); BOUVIER, Bernard, F-95610 Eragny (FR)
(74) Mandataire: Dronne, Guy
(86) Numéro de dépôt international: FR9600019
(87) Numéro de publication internationale: WO9621151

(56) Documents cités:
- EP-A- 0 053 453
- EP-A- 0 181 272
- EP-A- 0 222 986
- EP-A- 0 419 094
- EP-A- 0 495 538
- EP-A- 0 643 301
- WO-A-91/16087

## Description

La présente invention a pour objet un dispositif de contrôle de l'écoulement d'un liquide dans une conduite tubulaire.

Plus précisément, le dispositif de contrôle sert au moins à détecter la présence de bulles de gaz dans l'écoulement liquide, en particulier lorsque cette conduite tubulaire est la tubulure d'une pompe péristaltique.

Il existe un certain nombre de situations où il est important de s'assurer qu'un liquide circulant dans une conduite n'entraîne pas de façon accidentelle des bulles, notamment des bulles d'air. C'est en particulier le cas dans le domaine médical des conduites qui servent à amener le liquide de perfusion ou autre pour le malade.

Dans certaines circonstances, ce liquide est acheminé vers l'aiguille de perfusion dans une conduite, non pas par un simple effet de gravité, mais à l'aide d'une pompe, par exemple d'une pompe à galet ou pompe péristaltique. Une telle pompe est décrite notamment dans la demande de brevet français No. 2 691 258 déposée au nom de la demanderesse. Une telle disposition permet de contrôler de manière plus précise le débit d'injection du liquide. Dans le cas d'une pompe et quelle que soit sa précision de fonctionnement et l'étanchéité qu'elle présente, il peut toujours se trouver une quantité d'air inadmissible dans le liquide provenant d'une introduction accidentelle d'air dans le réservoir, d'une trop grande quantité d'air dissous dans le liquide ou éventuellement d'un effet de cavitation accidentelle provoquant l'entraînement de bulles d'air avec le liquide en circulation. On comprend bien sûr qu'une telle situation est totalement inacceptable et qu'il est particulièrement important de pouvoir détecter en permanence une éventuelle apparition de bulles d'air dans la conduite, notamment à la sortie de la pompe lorsqu'un tel dispositif est prévu afin de pouvoir interrompre l'admission du liquide.

Afin de détecter l'éventuelle présence de bulles d'air ou de gaz, on a déjà proposé des systèmes optiques de détection. C'est ce qui est décrit notamment dans la demande de brevet français déjà mentionnée. Cependant, cette détection optique n'est bien sûr utilisable que dans le cas où le liquide et la paroi de la conduite sont translucides ou sensiblement translucides.

Pour effectuer cette détection, on a également proposé d'utiliser des ultrasons dont on sait que le coefficient de transmission ou l'impédance de transmission varie selon la nature du fluide et varie donc selon que le liquide est dépourvu de bulles d'air ou bien au contraire qu'il comporte des bulles d'air. Dans les systèmes de détection par ultrasons connus, on utilise un émetteur et un récepteur qui sont constitués par des pastilles piézo-électriques disposées de part et d'autre de la tubulure, ces pastilles étant excitées en mode radial. Cette solution présente un premier inconvénient qui consiste dans le fait que, si la tubulure est placée dans un environnement lui-même liquide, la propagation des ultrasons du fait de l'émission peu directionnelle de la pastille piézo-électrique est perturbée entraînant un risque de laisser passer des bulles d'air sans les détecter. Un autre inconvénient de ces dispositifs émetteurs/récepteurs d'ultrasons est que, pour obtenir un faisceau d'ultrasons relativement directionnel, il est nécessaire d'utiliser des fréquences d'excitation relativement élevée et d'effectuer en conséquence une détection dans des signaux électriques également à fréquence élevée. Typiquement, ces fréquences sont supérieures à un Mégahertz et le plus souvent comprises entre 2 et 5 MHz. Il en résulte que l'environnement électrique et électronique de tels détecteurs est relativement complexe et donc onéreux.

Un objet de la présente invention est de fournir un dispositif de contrôle de l'écoulement d'un liquide dans une conduite, notamment pour la détection de bulles de gaz dans ce liquide, qui présente une grande fiabilité tout en étant d'un coût réduit et d'un encombrement limité.

Pour atteindre ce but, le dispositif de contrôle de l'écoulement d'un liquide dans une conduite tubulaire se caractérise en ce qu'il comprend :
- un bâti,
- une première pastille piézo-électrique dont l'axe de vibration est sensiblement orthogonal à ladite conduite dans la zone de détection et montée sur ledit bâti pour être disposée d'un premier côté de ladite conduite,
- des moyens pour exciter ladite première pastille piézo-électrique en mode axial à une fréquence prédéterminée F, afin d'émettre des ondes ultrasonores,
- une deuxième pastille piézo-électrique dont l'axe est sensiblement confondu avec celui de ladite première pastille et montée sur ledit bâti pour être disposée d'un deuxième côté de ladite tubulure, par quoi ladite deuxième pastille reçoit lesdites ondes ultrasonores ayant traversées ladite conduite,
- des moyens pour recueillir un signal électrique représentatif de l'amplitude de la vibration axiale de ladite deuxième pastille,
- des moyens de traitement dudit signal électrique pour en déduire la présence éventuelle d'une bulle de gaz dans le liquide circulant dans ladite conduite dans la zone de détection.

On comprend que, grâce à l'excitation en mode axial du capteur piézo-électrique et à la réception des vibrations également selon le mode axial d'excitation du récepteur, on obtient un faisceau d'ultrasons très directionnel et la fréquence d'excitation F peut être comprise entre 100 kHz et 1500 kHz selon la nature de la pastille piézo-électrique, ce qui est très sensiblement inférieur au système connu antérieurement. En conséquence, l'ensemble des circuits électriques et électroniques peut être considérablement simplifié.

De préférence, le bâti est celui d'une pompe péristaltique à galet et la conduite tubulaire est la portion de sortie de la tubulure déformable de ladite pompe.

De préférence, dans un plan perpendiculaire à l'axe de la tubulure, la dimension de la face émettrice/réceptrice de la pastille piézo-électrique est au plus égale à la hauteur de la lumière de la tubulure.

De préférence, encore, les deux pastilles piézo-électriques sont identiques.

De préférence également, dans l'application au contrôle du liquide délivré par une pompe du type péristaltique, le dispositif de contrôle comporte en même temps des moyens pour détecter une surpression accidentelle du liquide dans la tubulure de sortie, cette surpression entraînant la déformation de la tubulure.

Cette disposition est particulièrement intéressante puisqu'elle permet éventuellement d'arrêter le fonctionnement de la pompe en cas de difficulté d'écoulement du liquide, cette difficulté d'écoulement pouvant résulter soit d'une obturation accidentelle de la tubulure, soit d'une difficulté au niveau de l'aiguille de perfusion.

D'autres caractéristiques et avantages de la présente invention apparaîtront mieux à la lecture de la description qui suit de plusieurs modes de réalisation de l'invention donnés à titre d'exemples non limitatifs. La description se réfère aux figures annexées sur lesquelles :
- la figure 1 est une vue simplifiée d'un dispositif de détection de bulles par ultrasons,
- la figure 2 est une vue en coupe verticale d'un premier mode de réalisation d'un dispositif de détection de bulles appliqué au cas d'une pompe péristaltique, le dispositif de contrôle comportant en outre des moyens de détection d'une surpression dans la tubulure, et
- la figure 3 est une vue analogue à celle de la figure 2 montrant un deuxième mode de réalisation.

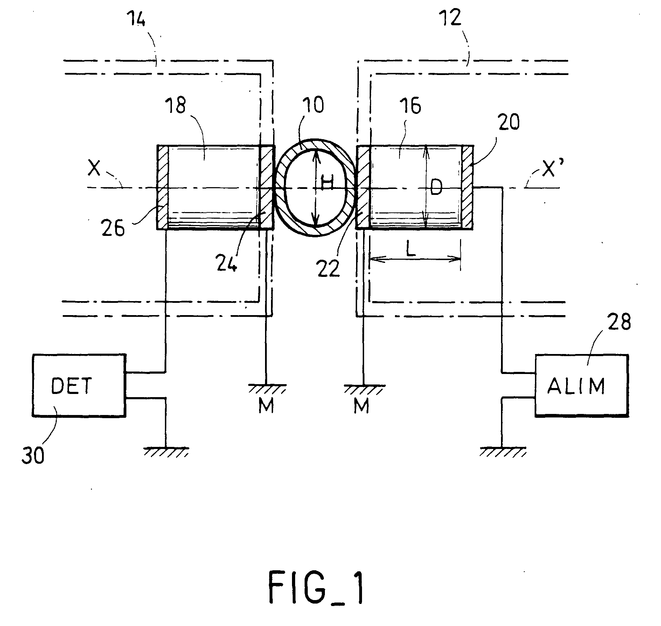

En se référant tout d'abord à la figure 1, on va décrire un mode de réalisation de l'invention permettant de détecter la présence éventuelle de bulles de gaz dans un liquide en écoulement dans une conduite.

Sur cette figure, on a représenté en section droite une tubulure 10 dans laquelle s'écoule le liquide. La tubulure 10 est disposée dans un bâti 12, 14 représenté schématiquement. De part et d'autre de la tubulure sont montées sur le bâti 12, 14 deux pastilles piézo-électriques respectivement référencées 16 et 18. Les pastilles piézo-électriques 16 et 18 sont du type à excitation axiale. Elles sont de forme générale cylindrique et présentent une longueur axiale L supérieure à leur diamètre D. A chaque extrémité terminale de la pastille 16 ou 18 sont réalisées des électrodes 20 et 22 pour la pastille émettrice 16 et 24 et 26 pour la pastille réceptrice 18. Les deux pastilles piézo-électriques présentent sensiblement un axe commun XX' qui est disposé dans le plan de section de la conduite 10 dans la zone de détéction. En outre, l'axe XX' est sensiblement orthogonal à l'axe de la conduite 10. Les électrodes 20 et 22 sont respectivement reliées à la masse M et à un générateur de signaux alternatifs 28 à fréquence contrôlée. On comprend qu'en appliquant aux électrodes 20 et 22 à l'aide du générateur d'impulsions 28 des impulsions électriques d'excitation à une fréquence F prédéterminée correspondant de préférence à la fréquence propre de la pastille piézo-électrique, celle-ci émet de façon directionnelle un faisceau d'ondes ultrasonores qui traversent la conduite 10 et dont le liquide circulant dans celle-ci. De préférence, la fréquence d'excitation de la pastille piézo-électrique, lorsqu'elle est réalisée en céramique, est comprise entre 100 et 1000 kHz ; typiquement elle est égale à 300 kHz. On comprend qu'ainsi, compte tenu de la fréquence relativement limitée, le générateur d'impulsions 28 et les circuits électriques associés peuvent être d'une construction relativement standard.

De préférence, les pastilles 16 et 18 sont identiques pour optimiser le couplage.

Symétriquement, la pastille piézo-électrique réceptrice 18 convertit sa fréquence de vibration axiale en un signal électrique à la même fréquence F, ce signal électrique étant recueilli par un détecteur 30. De tels détecteurs sont en soi connus. Ils sont de préférence synchrones de la fréquence d'excitation. Le détecteur en comparant le niveau de signal reçu à des seuils prédéterminés permet de détecter la présence éventuelle de bulles dans le liquide circulant dans la tubulure 10 par modification d'impédance acoustique.

Il faut de plus observer que, compte tenu du mode d'excitation des pastilles piézo-électriques 16 et 18, le faisceau d'ultrasons est très directionnel et la mesure n'est pas perturbée même si le dispositif de détection se retrouvait dans un milieu liquide.

Afin d'obtenir une détection optimale des bulles dans la tubulure 10, dans le plan de la figure, la dimension D de la pastille piézo-électrique 16 est au plus égale à la hauteur H de la lumière de la tubulure et de préférence sensiblement égale à celle-ci afin de détecter l'intégralité des éventuelles bulles de gaz.

Pour une tubulure ayant une hauteur H de 3 mm la pastille 16 en céramique a un diamètre de 2,5 mm et une longueur L de 5 mm. On peut également utiliser une pastille piézo-électrique composite stratifiée. Dans ce cas, la pastille a une section droite rectangulaire dont, par exemple, la hauteur est de 1,8 mm et la longueur (selon la direction perpendiculaire au plan de la figure) est égale à 4 mm. Son épaisseur est de 2,5 mm. La fréquence d'excitation en mode axial est comprise entre 200 et 1500 kHz et, de préférence, de l'ordre de 900 kHz.

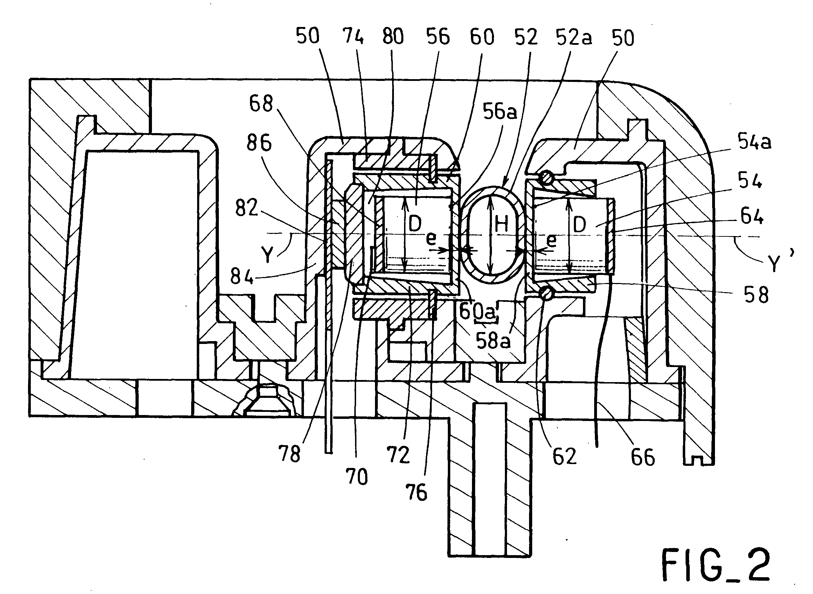

Après avoir décrit sous une forme simplifiée un mode de réalisation du dispositif de contrôle permettant la détection éventuelle de bulles de gaz dans le liquide, on va décrire en se référant à la figure 2 un premier mode complet de réalisation du dispositif de contrôle appliqué au cas d'une pompe péristaltique, ce dispositif permettant non seulement de détecter la présence éventuelle de bulles dans le liquide en circulation mais également de détecter une éventuelle surpression du liquide dans la conduite.

Sur la figure 2, on a représenté une partie du bâti 50 de la pompe péristaltique, ainsi que la portion de sortie 52 de la tubulure souple de la pompe péristaltique. Comme on l'a déjà expliqué en liaison avec la figure 1, le dispositif de détection de bulles est constitué par deux pastilles piézo-électriques excitées en mode axial référencées respectivement 54 et 56 qui sont disposées de part et d'autre de la tubulure 52 et dont les axes de vibration YY' sont sensiblement alignés et orthogonaux à l'axe de la tubulure 52 dans la zone de détection. Les deux -pastilles sont de préférence identiques et d'un des types décrits précédemment. Les faces avant 54a et 56a des pastilles semiconductrices sont fixées par tout moyen convenable sur respectivement les électrodes avant 58 et 60. Comme le montre la figure 2, les électrodes définissent une cavité qui va, de préférence, en s'évasant vers la tubulure. La face active de chaque pastille piézo-électrique est fixée sur la paroi mince 58a, 60a des électrodes fermant la cavité. Cette paroi mince, interposée entre la face active de la pastille et la conduite 52 a une épaisseur e inférieure à la demi-longueur d'onde des ondes ultrasonores émises par la pastille piézo-électrique. Pour la pastille céramique décrite précédemment, cette épaisseur est typiquement égale à 0,3 mm. De préférence, encore, la face externe de l'électrode 56 est recouverte par un revêtement du type "Téflon" afin d'assurer une isolation. Une autre solution consiste à réaliser les pièces 58 et 60 en un matériau isolant électrique tel que la bakélite, l'électrode et le conducteur électrique correspondant étant insérés dans la bakélite. Les électrodes 58 et 60 sont appliquées contre la face externe de la paroi 52a de la tubulure 52. Plus précisément, l'électrode 58 associée à la pastille piézo-électrique 54 est montée fixe dans le bâti 50 par l'intermédiaire d'un joint d'étanchéité 62. La deuxième électrode 64 de la pastille 54 est fixée sur sa deuxième face d'extrémité et reliée à un conducteur électrique 66. La pastille 56 réceptrice est également bien sûr munie d'une deuxième électrode 68 fixée sur sa deuxième face d'extrémité et reliée à un conducteur convenable 70. L'ensemble des pastilles piézo-électriques 54 et 56 permet d'effectuer la détection de la présence éventuelle de bulles dans le liquide circulant dans la conduite 52 selon le processus décrit en liaison avec la figure 1.

Afin de permettre de détecter de plus une éventuelle surpression du liquide dans la tubulure 52 et donc une déformation de la paroi souple 52a de cette tubulure, selon un mode préféré de mise en oeuvre de l'invention, l'électrode 60 est prolongée par une pièce formant manchon 72 qui entoure la pastille 56. Le manchon 72 est monté coulissant dans une pièce cylindrique 74 solidaire du bâti 50 de la pompe péristaltique. Une membrane souple élastique 76 assure une liaison mécanique entre l'électrode mobile 60 et le bâti de la pompe autorisant ainsi un déplacement de l'électrode 60 par rapport au bâti sous l'effet de la dilatation éventuelle de la tubulure 52. De plus, la membrane souple tend à appliquer l'ensemble mobile et donc la pastille piézo-électrique 56 contre la tubulure 52. Un capot 78 ferme la cavité 80 contenant la pastille piézo-électrique 56 pour maintenir l'étanchéité.

Pour détecter une éventuelle augmentation de pression, un capteur de pression, de préférence du type résistif, 82 est interposé entre une paroi fixe et rigide 84 du bâti et une pièce souple 86, cette pièce souple étant appliquée contre le capot 78 c'est-à-dire contre l'ensemble mobile contenant la pastille 56. Ainsi toute déformation de la tubulure 52 due à une surpression du liquide est transmise au capteur de pression 82 par l'ensemble mobile 60, 78 et la pièce déformable 86. De préférence, les faces des pièces 58 et 60 sont revêtues d'un matériau à très faible coefficient de frottement tel que le Téflon utilisé pour l'isolation des pastilles piézo-électriques. On évite ainsi l'introduction de contraintes parasites dans des directions autres que celle de l'axe X X'. Le capteur délivre alors un signal électrique qui, traité par des circuits de détection convenables, déclenche un signal d'alarme ou entraîne un arrêt automatique du fonctionnement de la pompe.

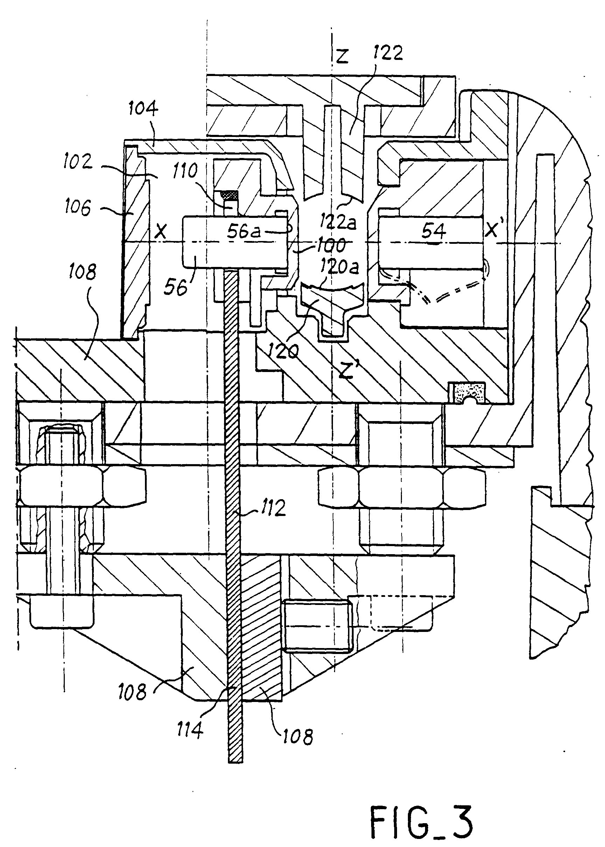

La figure 3 illustre une variante de réalisation du dispositif de détection de la figure 2. Selon cette variante, le montage du capteur piézo-électrique 54 est analogue à celui de la figure 2. Il ne sera donc pas décrit à nouveau. En revanche, le montage du capteur 56 associé au détecteur de surpression dans le tube 52 est modifié. On va le décrire ci-après.

La face active 56a du deuxième capteur 56 est fixée sur la pièce 100 qui est libre par rapport au logement 102 défini par les pièces 104 et 106 solidaires du boîtier principal 108. La pièce 100 et le piézo 56 sont solidaires de l'extrémité 110 d'une jauge de contrainte 112 travaillant en flexion. L'autre extrémité 114 de la jauge 112 est solidaire du boîtier 108, par exemple par encastrement. La jauge de contrainte 112 remplit une double fonction. D'autre part, par sa déformation en flexion, elle détecte une surpression dans la tubulure de la pompe, cette surpression entraînant un déplacement de la pièce 100 et du capteur 56 et donc de l'extrémité 110 du capteur. D'autre part, par son élasticité, la jauge 112 tend à appliquer en permanence la pièce 100 contre la tubulure de la pompe.

Pour assurer l'étanchéité du capteur 56, le logement 102 est rempli par un gel qui, après sa prise, est suffisamment "compact" pour rester dans le logement 102, mais qui n'entraîne aucune contrainte de frottement ou autres lorsque la pièce 100 se déplace sous l'effet des surpressions dans la tubulure de la pompe.

Pour améliorer encore la précision de la détection d'une surpression dans la tubulure, deux pièces 120 et 122 disposées à angle droit par rapport à l'axe des capteurs 54 et 56 sont solidaires de la cassette. Ces pièces qui présentent des faces 120a et 122a en forme de portions de surface cylindrique, empêche la déformation de la tubulure selon la direction orthogonale Z Z' et concentrent ainsi la déformation selon la direction "X X" des axes des capteurs et donc selon la direction de déplacement de l'extrémité 110 de la jauge de contrainte 112.

## Revendications

1. Dispositif de contrôle de l'écoulement d'un liquide dans une conduite tubulaire (10, 52), caractérisé en ce qu'il comprend :
- un bâti (12, 14, 50),
- une première pastille piézo-électrique (16, 54) dont l'axe de vibration est sensiblement orthogonal à ladite conduite dans la zone de détection et montée sur ledit bâti pour être disposée d'un premier côté de ladite conduite,
- des moyens (20, 22, 28, 58, 64) pour exciter ladite première pastille piézo-électrique en mode axial à une fréquence prédéterminée F inférieure à 1 MHz, afin d'émettre des ondes ultrasonores,
- une deuxième pastille piézo-électrique (18, 56) dont l'axe est sensiblement confondu avec celui de ladite première pastille et montée sur ledit bâti pour être disposée d'un deuxième côté de ladite tubulure, par quoi ladite deuxième pastille reçoit lesdites ondes ultrasonores ayant traversées ladite conduite, lesdites pastilles étant appliquées directement sur ladite conduite tubulaire,
- des moyens (24, 26, 60, 68) pour recueillir un signal électrique représentatif de l'amplitude de la vibration axiale de ladite deuxième pastille,
- des moyens de traitement (30) dudit signal électrique pour en déduire la présence éventuelle d'une bulle de gaz dans le liquide circulant dans ladite conduite dans la zone de détection.

2. Dispositif de contrôle de l'écoulement d'un liquide dans une conduite tubulaire (10, 52), caractérisé en ce qu'il comprend :
- un bâti (12, 14, 50),
- une première pastille piézo-électrique (16, 54) dont l'axe de vibration est sensiblement orthogonal à ladite conduite dans la zone de détection et montée sur ledit bâti pour être disposée d'un premier côté de ladite conduite, la face avant de ladite pastille étant recouverte d'un revêtement en matériau isolant,
- des moyens (20, 22, 28, 58, 64) pour exciter ladite première pastille piézo-électrique en mode axial à une fréquence prédéterminée F inférieure à 1 MHz, afin d'émettre des ondes ultrasonores,
- une deuxième pastille piézo-électrique (18, 56) dont l'axe est sensiblement confondu avec celui de ladite première pastille et montée sur ledit bâti pour être disposée d'un deuxième côté de ladite tubulure, par quoi ladite deuxième pastille reçoit lesdites ondes ultrasonores ayant traversées ladite conduite, la face avant de ladite pastille étant recouverte d'un revêtement en matériau isolant à très faible coefficient de frottement, lesdites pastilles équipées de leur revêtement étant directement appliquées sur ladite conduite tubulaire,
- des moyens (24, 26, 60, 68) pour recueillir un signal électrique représentatif de l'amplitude de la vibration axiale de ladite deuxième pastille,
- des moyens de traitement (30) dudit signal électrique pour en déduire la présence éventuelle d'une bulle de gaz dans le liquide circulant dans ladite conduite dans la zone de détection.

3. Dispositif de contrôle selon l'une quelconque des revendications 1 et 2, caractérisé en ce que les deux pastilles sont identiques.

4. Dispositif de contrôle selon l'une quelconque des revendications 1 à 3, caractérisé en ce que dans un plan perpendiculaire à l'axe de la conduite, la dimension de la face émettrice/réceptrice de la pastille piézo-électrique est au plus égale à la hauteur de la lumière de ladite conduite.

5. Dispositif de contrôle selon la revendication 4, caractérisé en ce que ladite dimension est sensiblement égale à la hauteur de la lumière de la conduite.

6. Dispositif de contrôle selon l'une quelconque des revendications 1 à 5, caractérisé en ce que ledit bâti (50) est celui d'une pompe péristaltique et la conduite tubulaire (52) est la portion de sortie de la tubulure déformable de ladite pompe.

7. Dispositif selon la revendication 6, caractérisé en ce que ladite deuxième pastille piézo-électrique (56) est solidaire d'un support mobile (60, 72, 78) en translation par rapport audit bâti selon la direction de son axe, en ce que ladite première pastille (54) est fixe par rapport audit bâti, et en ce qu'il comprend en outre des moyens élastiques (76) interposés entre ledit bâti et ledit support mobile tendant à appliquer avec pression ladite deuxième pastille contre la paroi de ladite conduite (52) et des moyens (82) de détection du déplacement dudit support mobile par rapport au bâti sous l'effet de la déformation de ladite conduite en raison d'une surpression du liquide circulant dans ladite conduite.

8. Dispositif selon la revendication 7, caractérisé en ce que les moyens de détection de déplacement comprennent un capteur de pression (82) interposé entre le bâti (50, 84) et une extrémité (78) dudit support mobile.

9. Dispositif selon la revendication 8, caractérisé en ce que ledit capteur de pression (82) est un capteur résistif.

10. Dispositif selon la revendication 6, caractérisé en ce que ladite deuxième pastille piézo-électrique (56) est solidaire d'un support mobile (100) en translation par rapport audit boîtier (108) selon la direction de son axe (X X'), en ce que ladite première pastille (54) est fixe par rapport audit bâti, et en ce qu'il comprend en outre une jauge de contrainte (112) travaillant en flexion dont une première extrémité (110) est solidaire dudit support mobile (100) et dont l'autre extrémité (114) est solidaire du bâti (108) par quoi ladite jauge de contrainte tend à appliquer ledit support (100) contre la conduite tubulaire (52) de ladite pompe et détecte les déplacements dudit support pour détecter une éventuelle surpression dans la conduite tubulaire.

11. Dispositif selon la revendication 10, caractérisé en ce que ledit support (100) et ladite deuxième pastille piézo-électrique sont montées dans un logement (102) du bâti (108), ledit logement étant rempli d'un gel.

12. Dispositif selon l'une quelconque des revendications 1 à 11, caractérisé en ce que lesdites pastilles sont réalisées en céramique et ladite fréquence prédéterminée est au moins égale à 100 kHz.

13. Dispositif selon la revendication 12, caractérisé en ce que ladite fréquence est sensiblement égale à 300 kHz.

14. Dispositif selon l'une quelconque des revendications 1 à 11, caractérisé en ce que lesdites pastilles sont réalisées en composite stratifié et ladite fréquence prédéterminée est au moins égale à 200 kHz.

15. Dispositif selon la revendication 14, caractérisé en ce que ladite fréquence est de l'ordre de 900 kHz.

## Claims

1. A monitoring device for monitoring the flow of a liquid in a tubular duct (10, 52), the device being characterized in that it comprises:
˙ a support structure (12, 14, 50);
˙ a first piezoelectric pellet (16, 54) whose vibration axis is substantially orthogonal to said duct in the detection zone and which is mounted on said support structure to be disposed on a first side of said duct;
˙ means (20, 22, 28, 58, 64) for exciting said first piezoelectric pellet in axial mode at a predetermined frequency F that is lower than 1 MHz, in order to transmit ultrasound waves;
˙ a second piezoelectric pellet (18, 56) whose axis coincides substantially with that of the first pellet and mounted on said support structure so as to be disposed on a second side of said tube, whereby said second pellet receives said ultrasound waves after they have passed through said duct, with said pellets being applied directly to said tubular duct;
˙ means (24, 26, 60, 68) for picking up an electric signal representative of the amplitude of axial vibration of said second pellet; and
˙ processor means (30) for processing said electric signal to deduce therefrom the presence of a gas bubble, if any, in the liquid flowing in said duct through the detection zone.

2. A monitoring device for monitoring the flow of a liquid in a tubular duct (10, 52), the device being characterized in that it comprises:
˙ a support structure (12, 14, 50);
˙ a first piezoelectric pellet (16, 54) whose vibration axis is substantially orthogonal to said duct in the detection zone and which is mounted on said support structure to be disposed on a first side of said duct, the front face of said pellet being covered with a coating made of insulating material;
˙ means (20, 22, 28, 58, 64) for exciting said first piezoelectric pellet in axial mode at a predetermined frequency F that is lower than 1 MHz, in order to transmit ultrasound waves;
˙ a second piezoelectric pellet (18, 56) whose axis coincides substantially with that of the first pellet and mounted on said support structure so as to be disposed on a second side of said tube, whereby said second pellet receives said ultrasound waves after they have passed through said duct, with the front face of said pellet being covered with a coating made of insulating material having a very low coefficient of friction and said thus coated pellets being directly applied to said tubular duct;
˙ means (24, 26, 60, 68) for picking up an electric signal representative of the amplitude of axial vibration of said second pellet; and
˙ processor means (30) for processing said electric signal to deduce therefrom the presence of a gas bubble, if any, in the liquid flowing in said duct through the detection zone.

3. A monitoring device according to any one of claims 1 and 2, characterized in that the two pellets are identical.

4. A monitoring device according to claim 1 to 3, characterized in that, in a plane perpendicular to the axis of the duct, the dimension of the transmitter/receiver face of the piezoelectric pellet is no greater than the height of the bore of said duct.

5. A monitoring device according to claim 4, characterized in that said dimension is substantially equal to the height of the bore of the duct.

6. A monitoring device according to any one of claims 1 to 5, characterized in that said support structure (50) is the support structure of a peristaltic pump, and the tubular duct (52) is the outlet portion of the deformable tube of said pump.

7. A device according to claim 6, characterized in that said second piezoelectric pellet (56) is secured to a moving support (60, 72, 78) that is movable in translation relative to said support structure along the direction of the axis of the pellet, in that said first pellet (54) is fixed relative to said support structure, and in that it further comprises resilient means (76) interposed between said support structure and said moving support tending to press said second pellet against the wall of said duct (52), and displacement detection means (82) for detecting the displacement of said moving support relative to the support structure under the effect of deformation of said duct because of excess pressure of the liquid flowing along said duct.

8. A device according to claim 7, characterized in that the displacement detection means comprise a pressure sensor (82) interposed between the support structure (50, 84) and one end (78) of said moving support.

9. A device according to claim 8, characterized in that said pressure sensor (82) is a resistive sensor.

10. A device according to claim 6, characterized in that said second piezoelectric pellet (56) is secured to a moving support (100) that is movable in translation relative to said support structure (108) in the direction of the axis (XX') of the pellet, in that said first pellet (54) is fixed relative to said support structure, and in that it further includes a strain gauge (112) operating in bending, having one end (110) secured to said moving support (100) and having its other end (114) secured to the support structure (108), whereby said strain gauge tends to press said support (100) against the tubular duct (52) of said pump, and detects the displacements of said support to detect any excess pressure in the tubular duct.

11. A device according to claim 10, characterized in that said support (100) and said second piezoelectric pellet are mounted in a housing (102) of the support structure (108), said housing being filled with a gel.

12. A device according to any one of claims 1 to 11, characterized in that said pellets are made of ceramic and said predetermined frequency is at least equal to 100 kHz.

13. A device according to claim 12, characterized in that said frequency is substantially equal to 300 kHz.

14. A device according to any one of claims 1 to 11, characterized in that said pellets are made of stratified composite, and said predetermined frequency is at least equal to 200 kHz.

15. A device according to claim 14, characterized in that said frequency is about 900 kHz.

## Patentansprüche

1. Vorrichtung zur Durchflusskontrolle einer Flüssigkeit in einer rohrförmigen Leitung (10, 52), dadurch gekennzeichnet, dass sie folgendes aufweist:
- einen Rahmen (12, 14, 50),
- einen ersten piezoelektrischen Chip (16, 54), dessen Schwingungsachse im wesentlichen senkrecht zur Leitung im Erfassungsbereich verläuft und welcher auf dem Rahmen so angebracht ist, dass er sich auf einer ersten Seite der Leitung befindet,
- Einrichtungen (20, 22, 28, 58, 64) zum Erregen des ersten piezoelektrischen Chips im Axialmodus mit einer vorgegebenen Frequenz F unter 1 MHz, damit dieser Ultraschallwellen aussendet,
- einen zweiten piezoelektrischen Chip (18, 56), dessen Achse im wesentlichen mit der Achse des ersten Chips zusammenfällt und welcher auf dem Rahmen so angebracht ist, dass er sich auf einer zweiten Seite der Rohrleitung befindet, wodurch der zweite Chip die Ultraschallwellen nach deren Durchgang durch die Leitung empfängt, wobei die Chips direkt auf die Rohrleitung aufgebracht sind,
- Einrichtungen (24, 26, 60, 68) zum Auffangen eines elektrischen Signals, das für die Amplitude der Schwingungen des zweiten Chips in axialer Richtung repräsentativ ist,
- Einrichtungen (30) zum Verarbeiten des elektrischen Signals in der Weise, dass daraus die eventuelle Anwesenheit einer Gasblase in der in der Leitung zirkulierenden Flüssigkeit im Erfassungsbereich abgeleitet wird.

2. Vorrichtung zur Durchflusskontrolle einer Flüssigkeit in einer rohrförmigen Leitung (10, 52), dadurch gekennzeichnet, dass sie folgendes aufweist:
- einen Rahmen (12, 14, 50),
- einen ersten piezoelektrischen Chip (16, 54), dessen Schwingungsachse im wesentlichen senkrecht zur Leitung im Erfassungsbereich verläuft und welcher auf dem Rahmen so angebracht ist, dass er sich auf einer ersten Seite der Leitung befindet, wobei die Vorderseite des Chips mit einem Überzug aus Isoliermaterial beschichtet ist,
- Einrichtungen (20, 22, 28, 58, 64) zum Erregen des ersten piezoelektrischen Chips im Axialmodus mit einer vorgegebenen Frequenz F unter 1 MHz, damit dieser Ultraschallwellen aussendet,
- einen zweiten piezoelektrischen Chip (18, 56), dessen Achse im wesentlichen mit der Achse des ersten Chips zusammenfällt und welcher auf dem Rahmen so angebracht ist, dass er sich auf einer zweiten Seite der Rohrleitung befindet, wodurch der zweite Chip die Ultraschallwellen nach deren Durchgang durch die Leitung empfängt, wobei die Vorderseite des Chips mit einem Überzug aus Isoliermaterial mit sehr kleinem Reibungskoeffizienten beschichtet ist, und wobei die Chips mit ihrem Überzug direkt auf die Rohrleitung aufgebracht sind,
- Einrichtungen (24, 26, 60, 68) zum Auffangen eines elektrischen Signals, das für die Amplitude der Schwingungen des zweiten Chips in axialer Richtung repräsentativ ist,
- Einrichtungen (30) zum Verarbeiten des elektrischen Signals in der Weise, dass daraus die eventuelle Anwesenheit einer Gasblase in der in der Leitung zirkulierenden Flüssigkeit im Erfassungsbereich abgeleitet wird.

3. Kontrollvorrichtung nach einem der Ansprüche 1 und 2, dadurch gekennzeichnet, dass die beiden Chips identisch sind.

4. Kontrollvorrichtung nach einem der Ansprüche 1 bis 3, dadurch gekennzeichnet, dass die Abmessung der Sendefläche/Empfangsfläche des piezoelektrischen Chips höchstens gleich der Höhe der Öffnung der Leitung ist.

5. Kontrollvorrichtung nach Anspruch 4, dadurch gekennzeichnet, dass die Abmessung im wesentlichen gleich der Höhe der Öffnung der Leitung ist.

6. Kontrollvorrichtung nach einem der Ansprüche 1 bis 5, dadurch gekennzeichnet, dass der Rahmen (50) der Rahmen einer peristaltischen Pumpe ist und dass die Rohrleitung (52) den Ausgangsabschnitt der verformbaren Rohrleitung der Pumpe bildet.

7. Vorrichtung nach Anspruch 6, dadurch gekennzeichnet, dass der zweite piezoelektrische Chip (56) fest mit einem beweglichen Träger (60, 72, 78) verbunden ist, der bezüglich des Rahmens in Richtung von dessen Achse translatorisch bewegbar ist, dass der erste Chip (54) bezüglich des Rahmens fest steht, und dass sie des weiteren elastische Einrichtungen (76) aufweist, die zwischen dem Rahmen und dem beweglichen Träger eingesetzt und bestrebt sind, den zweiten Chip gegen die Wandung der Leitung (52) unter Druck anzulegen, sowie Einrichtungen (82) zum Erfassen der Verlagerung des beweglichen Trägers bezüglich des Rahmens unter der Einwirkung der Verformung der Leitung infolge eines Überdrucks der in der Leitung zirkulierenden Flüssigkeit.

8. Vorrichtung nach Anspruch 7, dadurch gekennzeichnet, dass die Einrichtungen zum Erfassen der Verlagerung einen zwischen dem Rahmen (50, 84) und einem Ende (78) des beweglichen Trägers eingesetzten Druckfühler (82) umfassen.

9. Vorrichtung nach Anspruch 8, dadurch gekennzeichnet, dass der Druckfühler (82) ein Widerstandssensor ist.

10. Vorrichtung nach Anspruch 6, dadurch gekennzeichnet, dass der zweite piezoelektrische Chip (56) fest mit einem beweglichen Träger (100) verbunden ist, welcher bezüglich des Gehäuses (108) entlang dessen Achse (X-X') translatorisch bewegbar ist, dass der erste Chip (54) bezüglich des Rahmens fest steht, und dass sie des weiteren ein mit Biegung funktionierendes Dehnungsmessmittel (112) aufweist, dessen eines Ende (110) fest mit dem beweglichen Träger (100) verbunden ist und dessen anderes Ende (114) fest mit dem Rahmen (108) verbunden ist, so dass das Dehnungsmessmittel bestrebt ist, den Träger (100) gegen die Rohrleitung (52) der Pumpe anzulegen und die Verlagerungen des Trägers erfasst, um so einen eventuellen Überdruck in der Rohrleitung zu erfassen.

11. Vorrichtung nach Anspruch 10, dadurch gekennzeichnet, dass der Träger (100) und der zweite piezoelektrische Chip in einer Aufnahme (102) im Rahmen (108) angebracht sind, wobei die Aufnahme mit einem Gel gefüllt ist.

12. Vorrichtung nach einem der Ansprüche 1 bis 11, dadurch gekennzeichnet, dass die Chips aus Keramikmaterial gefertigt sind und dass die vorgegebene Frequenz mindestens gleich 100 kHz ist.

13. Vorrichtung nach Anspruch 12, dadurch gekennzeichnet, dass die Frequenz im wesentlichen gleich 300 kHz ist.

14. Vorrichtung nach einem der Ansprüche 1 bis 11, dadurch gekennzeichnet, dass die Chips aus einem Schichtverbundstoff bestehen und dass die vorgegebene Frequenz im wesentlichen gleich 200 kHz ist.

15. Vorrichtung nach Anspruch 14, dadurch gekennzeichnet, dass die Frequenz im Größenbereich von 900 kHz liegt.
